(19)

Europäisches Patentamt
European Patent Office
Office européen des brevets

(11) **EP 4 153 996 B1**

(12) **EUROPEAN PATENT SPECIFICATION**

(45) Date of publication and mention
of the grant of the patent:
**30.04.2025  Bulletin 2025/18**

(21) Application number: **21730644.8**

(22) Date of filing: **21.05.2021**

(51) International Patent Classification (IPC):
**G01N 33/52** *(2006.01)*      **G01N 27/447** *(2006.01)*
**G01N 33/68** *(2006.01)*

(52) Cooperative Patent Classification (CPC):
**G01N 33/52; G01N 27/44791; G01N 33/6845;**
G01N 2030/8831

(86) International application number:
**PCT/GB2021/051244**

(87) International publication number:
**WO 2021/234411 (25.11.2021 Gazette 2021/47)**

(54) **IMPROVEMENTS IN OR RELATING TO AN APPARATUS FOR CHARACTERISING A COMPONENT**

VERBESSERUNGEN AN ODER IM ZUSAMMENHANG MIT EINER VORRICHTUNG ZUR CHARAKTERISIERUNG EINER KOMPONENTE

AMELIORATIONS DE OU RELATIVES À UN APPAREIL DE CARACTERISATION UN COMPOSANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **22.05.2020  GB 202007690**

(43) Date of publication of application:
**29.03.2023  Bulletin 2023/13**

(73) Proprietors:
• **Fluidic Sciences Limited**
  **Royston, Hertfordshire SG8 5HW (GB)**
• **Cambridge Enterprise Limited**
  **Cambridge, Cambridgeshire CB2 1TN (GB)**

(72) Inventors:
• **DEVENISH, Sean**
  **Cambridge CB1 8DH (GB)**
• **KNOWLES, Tuomas**
  **Cambridge CB2 1EW (GB)**
• **KOSMOLIAPTSIS, Vasilis**
  **Cambridge CB2 0QQ (GB)**
• **MEISL, Georg**
  **Cambridge CB2 1EW (GB)**
• **PRIDDEY, Ashley**
  **Cambridge CB2 0QQ (GB)**

• **SCHEIDT, Tom**
  **Cambridge CB2 1EW (GB)**
• **SCHNEIDER, Matthias**
  **Cambridge CB2 1EW (GB)**
• **XU, Catherine**
  **Cambridge CB2 1EW (GB)**

(74) Representative: **Stratagem IPM Limited**
**Meridian Court**
**Comberton Road**
**Toft, Cambridge CB23 2RY (GB)**

(56) References cited:
**WO-A1-2015/071683**

• **LÓPEZ PUGA JORGE: "Bayesian statistics", 29 April 2015 (2015-04-29), pages 377 - 379, XP055832497, Retrieved from the Internet <URL:https://www.nature.com/articles/ nmeth.3368.pdf> [retrieved on 20210817]**
• **AROSIO PAOLO ET AL: "Microfluidic Diffusion Analysis of the Sizes and Interactions of Proteins under Native Solution Conditions", ACS NANO, vol. 10, no. 1, 26 January 2016 (2016-01-26), US, pages 333 - 341, XP055832498, ISSN: 1936-0851, Retrieved from the Internet <URL:https://pubs. acs.org/doi/pdf/10.1021/acsnano.5b04713> DOI: 10.1021/acsnano.5b04713**

- YUEWEN ZHANG ET AL: "Top-down proteomics on a microfluidic platform", ARXIV.ORG, CORNELL UNIVERSITY LIBRARY, 201 OLIN LIBRARY CORNELL UNIVERSITY ITHACA, NY 14853, 25 October 2019 (2019-10-25), XP081521177
- FIEDLER SEBASTIAN ET AL: "Messung der Bindungsaffinität von Protein-Protein-Interaktionen", BIOSPEKTRUM, SPEKTRUM AKADEMISCHER VERLAG, DE, vol. 25, no. 7, 1 November 2019 (2019-11-01), pages 754 - 755, XP036958219, ISSN: 0947-0867, [retrieved on 20191204], DOI: 10.1007/S12268-019-1309-2

**Description**

The present invention relates to apparatus for characterising a component, such as a biomolecule, present within a liquid sample. In particular, the present invention relates to an apparatus configured to determine at least two characteristics of the component simultaneously.

Protein-protein interactions form the basis of many biologically and physiologically relevant processes including: protein self-assembly; protein-aggregation; antibody-antigen recognition; muscle contraction and cellular communication. Nevertheless, studying protein-protein interactions, especially under physiological conditions in complex media, remains challenging. Current techniques, such as an enzyme-linked immunosorbent assay (ELISA) bead-based multiplex assay and surface plasmon resonance (SPR) spectroscopy, rely on immobilisation of one binding partner. These techniques include potential unspecific interactions with the surface, which can cause false positive results and the Hook/Prozone effect, which causes false-negative results, thereby allowing semi-quantitative analysis only.

Of the more than 4500 proteins abundant in human serum, many are considered as potential biomarkers. Such biomarkers are not only useful tools in diagnosis of many diseases, including cancer, cardiovascular diseases, protein misfolding diseases, type-2-diabetes, mental disorders, and validation of drug trials, but are also proposed to be important indicators for auto-immune diseases and graft rejection. Assessment of biomarker levels requires a quantitative and reproducible procedure for absolute quantification, especially for long time evaluation. The absolute abundance and binding properties of distinct biomolecules correlate with the prevalence of certain diseases or act as hallmarks for potential immunological risk. Absolute quantification in human samples would not only streamline the finding of new biomarkers for diagnostics, but also help to gain understanding of detrimental processes through enabling correlation of concentration and affinity to the occurrence of an immune response.

As an example of a biomarker with clinical significance, the binding behaviour of antibodies against human leukocyte antigens (HLA), also known as the human major histocompatibility complex (MHC), has been identified as a suitable biomarker for numerous studies. The characteristic and unique structure of HLA facilitates the recognition of multiple pathogens. As such, HLA is involved in the distinction between self and foreign or malignant entities such as pathogens or extrinsic cells and tissues. As HLA is crucial in recognition of foreign organs and associated with allograft rejection, histocompatibility screening prior to organ transplantation is essential.

Currently all known approaches for clinical protein detection and quantification involve assays relying on surface immobilisation or bead immobilisation, as for example ELISA assays. While ELISA assays can achieve a relatively high sensitivity (usually down to fmol in ca. 100 μL well corresponding to 10,000-100,000 molecules), it is not capable of determining biophysical parameters describing the binding interaction in solution, although the physiologically relevant processes take place in solution.

Recently, alternative strategies have emerged for achieving enhanced sensitivity including the use an antibody pair for detecting protein molecules and the use of apparatus developed for flow cytometry for reading out relative intensities. However, these assays are still performed on the surface of a bead and therefore encounter the usual disadvantages of surface-based techniques described above.

Thus, there is a requirement to provide an in-solution platform that can be used to detect and determine specific concentrations, affinities, and the exact interaction stoichiometry of the interaction between biomarkers and their specific targets in body fluids in a fully quantitative manner.

It is against the background that the present invention has arisen.

According to an aspect of the present invention, there is provided an apparatus for characterising a biomolecule, the apparatus comprising a sample inlet channel configured to introduce a sample fluid including the biomolecule to the apparatus, an auxiliary inlet channel configured to introduce an auxiliary fluid to the apparatus, a distribution channel in fluid communication with the sample inlet channel and the auxiliary inlet channel; wherein the distribution channel is adapted to generate a lateral distribution of the biomolecule; a measurement module configured to detect a signature profile of the biomolecule to obtain a measured dataset of the detected biomolecule; wherein the measured dataset includes one or more of the following: auto fluorescence background measurements, flow profile, concentration, stoichiometry interactions, molecular weight of interacting biomolecules, total ligand concentration, binding sites, size, charge, modification such as post translational modification and/or the hydrodynamic radius; a storage location configured to store and maintain a stored dataset comprising a plurality of parameters that are associated with the measured dataset obtained from the measurement module; and an analysis module configured to receive the stored dataset from the storage location and correlate the stored dataset with the measured dataset from the measurement module to provide a correlation value, wherein the analysis module is further configured to use the correlation value to determine at least two characteristics of the biomolecule simultaneously using Bayesian analysis; and wherein the at least two characteristics are the concentration and the affinity or avidity of the biomolecule.

According to another aspect there is provided an apparatus for characterising a biomolecule, the apparatus comprising: a sample inlet channel configured to introduce a sample fluid including the biomolecule to the apparatus; an auxiliary inlet channel configured to introduce an auxiliary fluid to the apparatus; a distribution channel in fluid communication with the sample inlet channel and the auxiliary inlet channel; wherein the distribution channel is adapted to generate a distribution of biomolecules; a measurement module configured to detect a signature profile of the biomolecule to obtain a measured dataset of the detected biomolecule; a storage location configured to store and maintain a stored dataset comprising a plurality of parameters that are associated with the measured dataset obtained from the measurement module; and an analysis module configured to receive the stored dataset from the storage location and correlate the stored dataset with the measured dataset from the measurement module to provide a correlation value, wherein the analysis module is further configured to use the correlation value to determine at least two characteristics of the biomolecule simultaneously using Bayesian analysis.

The apparatus of the present invention allows for the determination of biophysical binding parameters in human samples such as human serum samples. The technique as disclosed herein allows for the determination of the dissociation constant or affinity $K_d$, the absolute concentration of the antibody present in the sample, as well as the stoichiometry of the interaction.

By using global analysis with varying the concentration of the serum as well as the concentration of the labelled antigen, it becomes possible to refine the characterisation using Bayesian Analysis.

In some cases, it can be highly advantageous to detect and measure two characteristics of each biomolecule simultaneously because it provides further granularity of information on the properties of the biomolecule which are not obtainable from known measurement techniques such as ELISA. For example, the apparatus can be used to detect and determine the concentration and the affinity of a biomolecule at the same time. Additionally or alternatively, the apparatus can be used to detect and determine the concentration and the avidity of a biomolecule at the same time.

The apparatus of the present invention may allow for the detection and determination of the biomolecule and its binding partner in solution. In some embodiments, biomolecule-biomolecule interactions may be characterised using the apparatus of the present invention. In some embodiments, the apparatus can be used to detect and determine the bound and/or unbound biomolecule. The relative concentration of both the biomolecule and of its binding partner can be varied. When biomolecules are bound to a surface the user is not able to freely vary the concentration of the bound biomolecule.

Furthermore, biomolecules immobilised onto a surface may not truly reflect their native state as an immobilisation species such as a chemical linker is often attached onto the biomolecule in order to immobilise the species onto the surface. This may affect the natural state or functional activity of the biomolecule immobilised on the surface. The apparatus as described in the present invention does not require an immobilisation technique of a biomolecule onto a surface to study binding interactions for example. Hence, the interactions between biomolecules can be determined and detected in solution and in their native state.

In some embodiments, the apparatus can be used to characterise a component such as a biomolecule where the biomolecule is a protein, a peptide, an exosome, an antibody or an antibody fragment thereof, a nucleotide such as DNA or DNA piece, RNA or mRNA, or a polysaccharide.

In some embodiments, the biomolecule is an antibody, a polypeptide, a polynucleotide or a polysaccharide. In some embodiments, the biomolecule is an antibody or an antibody fragment thereof. In some embodiments, the antibody is an allo-antibody, an autoantibody or an antibody raised against an external antigen. The term "allo-antibody" in this context is used to refer to an antibody that recognises foreign molecules, such as HLA, within the field of organ transplantation.

In some embodiments, the biomolecule is a multi-biomolecule mixture. In some embodiments, the biomolecule may be an affinity reagent such as an antibody, a single domain antibody or an aptamer. In some embodiments, the multi-biomolecule mixture comprises an antibody and an antigen. In some embodiments, one biomolecule in the multi-biomolecule mixture can be labelled or at least two or more biomolecules can be labelled. In some embodiments, the antibody can be labelled in order to detect other biomolecules of interest. In some cases, due to the fact that the antibody of interest may be mixed with other antibodies in a sample, it is preferable for the antigen to be labelled.

In some embodiments, the sample fluid can be any type of human fluid or sample. In some embodiments, the sample fluid can be a human serum comprising the biomolecule.

Bayesian analysis can be advantageous as it can be used to quantify both the affinity and the antibody concentration of a sample such as a human serum sample simultaneously with great accuracy and in a robust manner.

In some embodiments, the measured dataset may include one or more of the following, but is not limited to, auto fluorescence background measurements, flow profile, concentration of a labelled sample such as a labelled serum sample, the stoichiometry interactions, the molecular weight of interacting biomolecules, total ligand concentration, binding sites, size, charge, modification such as post translational modification for example methylation and/or the hydrodynamic radius.

In some embodiments, a microfluidic platform can be used as an in-solution technique capable of detecting and quantifying molecules in body fluids absolutely. More specifically, the platform is configured to quantify the abundance and affinity of antibodies in human serum. Thereby, an antigen against which the antibodies are reactive is added externally, and the change in hydrodynamic radius of the complex is monitored as a function of the serum concentration.

In some embodiments, the apparatus further comprises a controller configured to receive the stored dataset from the storage location and to receive the measured dataset from the analysis module to further tune one or more parameters associated with the measured dataset; and an output module associated with the controller configured to provide a notification to an operator indicating a further cycle of measurements such that further measurements obtained provide a pre-determined level of confidence set by a user in the determined characteristics of the biomolecule.

The level of confidence may involve statistical error analysis associated with the measurements obtained. The level of confidence can be set by the user as a pre-determined threshold value. If the threshold value or level is not reached as a result of an initial set of measurements, a further set of data is obtained to increase the level of confidence until it reaches the pre-determined threshold value or level set by the user. In some embodiments, the level of confidence may take into account the interactions between the biomolecules within the sample such as the interactions between the antibody and antigen in a human serum sample for instance.

The apparatus and method of the present invention provides an iterative process. Earlier measurements can be fed back into the controller in order to optimise one or more parameters associated with the measured dataset. Since the controller is associated with the output module, the output module is able to notify the operator an indication of further cycle of measurements. This enables the measurements to be taken in the most information rich part of the dataset. This, in turn, provides an increased level of confidence in the determined characteristics of the biomolecule such as affinity and concentration. Hence, this reduces the amount of time required to obtain further measurements of affinity and concentration of biomolecules.

In some embodiments, the level of confidence can be determined using statistical analysis to set a pre-determined threshold value. In some embodiments, the level of confidence value can be set by a user to be within an acceptable statistical range and/or standard deviation.

In some embodiments, the apparatus may further comprise the use of microfluidic diffusion analysis (MDS) to determine and analyse the amount of interaction between human leukocyte antigen (HLA) and allo-specific antibodies in patient sera and more particularly, in order to assess the risk of allograft rejection. Using this approach, it is possible to determine dissociation constants, $K_d$, the absolute concentration of the allo-specific antibody and the stoichiometry of the interaction at the same time.

In some embodiments, the concentration of the molecules that can be characterized using the apparatus of the present invention is unknown and can be determined through the use of a different data fitting method such as Bayesian analysis to both determine the fraction of serum and the concentration of HLA required to obtain measurements and to analyse these measurements.

Furthermore, the apparatus and method as described herein can be more widely applicable and describe it as a way of being able to profile the immune status of patients, for example, by being able to detect different antibodies in auto immune diseases.

In some embodiments, the distribution channel is adapted to generate a lateral distribution of biomolecule. The distribution channel may be of sufficient length/width to enable lateral distribution by diffusion. In some embodiments, the distribution channel may have a length of between 2 mm and 100 mm, for example 20 mm. In some embodiments, the distribution channel may have a length of more than 2, 10, 20, 30, 40, 50, 60, 70, 80 or 90 mm, or it may be less than 100, 90, 80, 70, 60, 50, 40, 30, 20 or 10 mm. The distribution channel may have a width of between 30 microns and 3,000 microns, for example between 20 and 2500 microns, 20 and 2000 microns, 20 and 1500 microns, 20 and 1000 microns, 20 and 750 microns, 20 and 500 microns, 20 and 400 microns or 20 microns and 300 microns. In some embodiments, the width of the distribution channel may exceed 20, 40, 60, 80, 100, 120, 150, 180, 200, 220, 250 or 280 microns, or it may be less than 300, 280, 250, 220, 200, 180, 150, 120, 100, 80, 60 or 40 microns. For example, the distribution channel may have a width of 40 x 40 microns and a length of 23 mm. In a further example, the distribution channel may have a width of between 50 x 70 microns and a length of 10 mm.

Alternatively, the distribution channel can be "adapted" by having an electric field spanning it to enable distribution of components by electrophoresis. Alternatively, the distribution channel can be adapted by providing a heat source which is configured to enable thermophoresis to distribute biomolecules.

In some embodiments, the lateral distribution of the biomolecule is perpendicular to the fluid flow along the distribution channel.

In some embodiments, the distribution channel may be adapted to generate a longitudinal distribution of a biomolecule. In such embodiments, the distribution of the biomolecules is parallel to (i.e. in direction of) the flow through the distribution channel. The distribution of the biomolecules may take place under steady state conditions or, conversely, under flow conditions.

In some embodiments, the apparatus may further comprise at least two outlet channels to divide the fluid in the distribution channel into two or more flows. At least one of the outlet channels may comprise a measurement zone where the measurement module is able to detect and measure one or more signature profiles of the biomolecule.

In some embodiments, the analysis module can be configured to detect and determine the concentration and the affinity of each biomolecule. The concentration and affinity measurements can provide useful information on how the biomolecule behaviours individually or in complex with other components for example in protein-protein interactions.

In some embodiments, the analysis module may be configured to detect and determine the concentration and the avidity of each biomolecule. As disclosed herein and unless otherwise defined, the term "avidity" refers to the overall binding strength of the interactions between components such as biomolecules when there is more than one binding site. As an example, the binding strength of the interactions between an antibody and an antigen can be determined. In some embodiments, the overall binding strength of the antibody-HLA interaction can be determined. The term "avidity" is applicable to quantifying the binding of multivalent biomolecules which have multiple binding sites. A measurement of the avidity takes into consideration the interaction at all available binding sites. As an example, the avidity of a biomolecule with at least two binding sites such as IgG, IgD or IgE can be quantified.

As disclosed herein and unless otherwise defined, the term "affinity" refers to the binding measurement of a component such as a biomolecule at one binding site.

Optionally, the analysis module may be configured to determine one or more of the following: flow profile, concentration of a labelled sample such as a labelled serum sample, the stoichiometry interactions, the molecular weight of interacting biomolecules, total ligand concentration, binding sites, size, charge, modification such as post translational modification for example methylation and/or the hydrodynamic radius.

In some embodiments, the analysis module may further be configured to detect and determine the stoichiometry of each biomolecule.

By using the apparatus as disclosed in the present invention, specific concentrations, affinities, and the exact interaction stoichiometry of the interaction between biomarkers and their specific targets in body fluids can be detectable in a fully quantitative manner.

In some embodiments, the distribution channel may be a T-sensor. In some embodiments, the distribution channel, together with the sample inlet channel, an auxiliary inlet channel and the outlet channels may form an H-filter. In the context of this specification, the term T-sensor is used to describe a distribution channel with exactly two inlets and the term H-filter is a distribution channel with exactly two inlets and exactly two outlets. Although some T-sensors and H-filters have 180° between their inlets this is not essential.

In some embodiments, the biomolecule comprises a label such as a fluorophore, a quantum dot or a nanoparticle. In some embodiments, the biomolecule is labelled with a fluorophore. The fluorophore enables the user to visually detect the biomolecule of interest. An example of a fluorophore is Alexafluor® 647 fluorophore. Other examples of suitable fluorophore may include, but is not limited to, Alexa Fluor™, ATTO, DyLight or other families exemplified by, but not limited to, individual dyes such as DyLight 350, ATTO 488, DY-489XL, Alexa Fluor™ 647 or Alexa Fluor™ 700. Dyes are not restricted to visible wavelength fluorescence, and may be active in the UV, visible or IR regions of the spectrum.

The label selected can provide a reliable and stable labelling strategy for fluorescence detection.

In some embodiments, a labelling strategy can be deployed for specific labelling of HLA and controlling the stoichiometry of the antibody-HLA interaction. In some embodiments, HLA can be covalently labelled with a fluorescence dye such as Alexafluor® 647. This ensures that the protein autofluorescence (tryptophan fluorescence) does not interfere with detection as the complexity of the autofluorescence from the sample can make it difficult to distinguish between the different proteins abundant in the serum.

In some embodiments, a correction for autofluorescence may be required in human serum. The background contribution of serum can be corrected by subtracting the background intensity from the measured intensities, allowing the correction of all binding curves in measurements. Hence, a standard curve of the fluorescence background may be needed for each sample.

In some embodiments, the fluorophore selected for labelling the biomolecule is in the far-red spectral region.

A fluorophore in the far-red spectral region can be advantageous at around $\lambda_{em,max}$ = 665 nm wavelength, since there is a reduced autofluorescence of human serum in this region. This means that there is less background interference for detecting biomolecules.

In some embodiments, an electrode is provided at the upstream of the distribution channel and an electrode is provided at the downstream of the distribution channel, so that, together, these electrodes apply an electric field across the distribution channel, perpendicular to the direction of the flow along the distribution channel. In such embodiments, the distribution of the component may occur by free flow electrophoresis (FFE).

The provision of an electric field will enable separation/distribution of the components for example, a biomolecule to occur by electrokinetic separation techniques such as capillary zone electrophoresis (CE) capillary gel electrophoresis (CGE), capillary isoelectric focusing (CIEF), capillary isotachophoresis and micellar electrokinetic chromatography (MEKC) or via an enrichment process or a blood lysis procedure.

In another aspect, there is provided a method for characterising a biomolecule, the method comprising the steps of:

introducing a sample fluid flow into a sample inlet channel;
introducing an auxiliary fluid flow into an auxiliary inlet channel;
contacting the sample fluid flow and the auxiliary fluid flow in a distribution channel to create a laminar fluid flow;

generating a distribution of biomolecules between contacting sample and auxiliary fluid;

detecting a signature profile of the biomolecule using a measurement module to obtain a measured dataset of the detected biomolecule;

providing a storage location configured to store and maintain a stored dataset comprising a plurality of parameters that are associated with the measured dataset obtained from the measurement module;

providing an analysis module configured to receive the stored dataset from the storage location and correlate the stored dataset with the measured dataset from the measurement module to provide a correlation value; and

utilising the correlation value to determine at least two characteristics of the biomolecule simultaneously using Bayesian analysis.

In some embodiments, the method may further comprise the steps of:

providing a controller configured to receive the stored dataset from the storage location and to receive the measured dataset from the analysis module to further tune one or more parameters associated with the measured dataset; and

notifying an operator using an output module associated with the controller to indicate a further cycle of measurements such that further measurements obtained provide a pre-determined level of confidence in the determined characteristics of the biomolecule.

The output module can be a reader device or it can be any other device capable of displaying data to the operator.

In some embodiments, the lateral distribution is created by diffusion. Additionally or alternatively, the lateral distribution is created electrophoretically through the application of an electric field.

Additionally or alternatively, the measurement of the biomolecule is determined by microfluidic diffusional sizing (MDS), which is used to measure the size of the biomolecule based on the degree to which they diffuse within a fluid flow. In some embodiments, the apparatus and method as disclosed in the present invention may rely on MDS, which is capable of determining concentrations, affinities and stoichiometry of interactions in body fluids, and is, thus, able to shed light on the relevance of those biophysical properties for clinical assessment.

The sensitivity of the MDS platform is high i.e. it provides a good signal to noise ratio and therefore, it is a suitable technique for characterising biomolecule interactions in a sample at concentration in the low nM range.

MDS can also be used for the determination of binding parameters by utilising the measured hydrodynamic radius, Rh, of a fluorescently labelled protein by tracking its spatial and temporal evolution in a microfluidic channel under laminar flow. Moreover, MDS can be used as an advanced technique to quantify an analyte in human serum under native solution conditions, thereby yielding physiological relevant results with further clinical implications.

In addition, the use of MDS on body fluids avoids the requirement for a washing step and therefore the apparatus and method as described in the present invention can avoid sample loss and reducing false negative effects. This is in contrast to surface assays which require a washing step to take place and hence can result in sample loss and false negative effects.

Additionally or alternatively, the lateral distribution can be created by thermophoresis via the application of heat. In such embodiments, the apparatus may further comprise a heat source.

In some embodiments, the analysis module may be configured to detect and determine the concentration and the affinity and/or the avidity of each biomolecule.

In some embodiments, the analysis module may be further configured to detect and determine the stoichiometry of each biomolecule.

In some embodiments, the method may further comprise the step of detecting the biomolecule using fluorescence spectroscopy.

The invention will now be further and more particularly described, by way of example only, and with reference to the accompanying drawings, in which:

Figure 1 shows an apparatus for characterising a biomolecule according to the present invention;

Figures 2A to 2C provide plots showing binding experiments of HLA;

Figures 3A to 3D show binding curves for HLA against an antibody in human serum;

Figures 4A to 4D show data on testing of human patient serum against different HLA variants;

Figures 5 and 5B provide a labelling strategy of the sample according to Figure 1;

Figure 6A and Figure 6B provide a further labelling strategy of the sample according to Figure 1;

Figure 7 shows a binding curve for interaction;

Figures 8A to 8E show data of covalent labelling according to the present invention;

Figures 9A to 9F show the determination of the absorbance of varying concentration of a label in human serum;

Figure 10 shows the structure of bilirubin;

Figures 11A and 11B show plots of negative control experiments;

Figure 12 shows a further plot of a negative control experiment;

Figures 13A and 13B show global fit for the binding curves for interactions between HLA and an antibody;

Figures 14A and 14B shows the binding curve of human serum against HLA; and

Figure 15 showing an affinity plot of serum antibody reactivity against the Receptor Binding Domain (RBD) of SARS-CoV-2 Spike protein.

According to the present invention, there is provided an apparatus for characterising a biomolecule and in some cases the apparatus can be used to characterise biomolecule interactions such as antibody-antigen interactions. The apparatus comprises a sample inlet channel configured to introduce a sample fluid including the biomolecule to the apparatus, an auxiliary inlet channel configured to introduce an auxiliary fluid to the apparatus and a distribution channel which is in fluid communication with the sample inlet channel and the auxiliary inlet channel. The distribution channel is adapted to generate a distribution of biomolecules through diffusion such as lateral diffusion and/or through electrophoresis upon the application of an electric field or through thermophoresis upon the application of heat. Additionally or alternatively, the distribution of biomolecules within the distribution channel can be generated via microfluidic diffusional sizing (MDS).

In utilising the MDS assay for absolute quantification and characterisation of molecules in solution, it is possible to determine the hydrodynamic radius, Rh, in human serum and hence, it is possible to characterise the interactions of biomolecules in measured in human serum. This is because MDS has been show to allow the determination of binding parameters by utilising the measured hydrodynamic radius, Rh, of a fluorescently labelled protein by tracking its spatial and temporal evolution in a microfluidic channel under laminar flow.

The apparatus further comprises a measurement module configured to detect a signature profile of the biomolecule to obtain a measured dataset of the detected biomolecule; a storage location configured to store and maintain a stored dataset comprising a plurality of parameters that are associated with the measured dataset obtained from the measurement module; and an analysis module configured to receive the stored dataset from the storage location and correlate the stored dataset with the measured dataset from the measurement module to provide a correlation value. The analysis module is further configured to use the correlation value to determine at least two characteristics of the biomolecule simultaneously using Bayesian analysis.

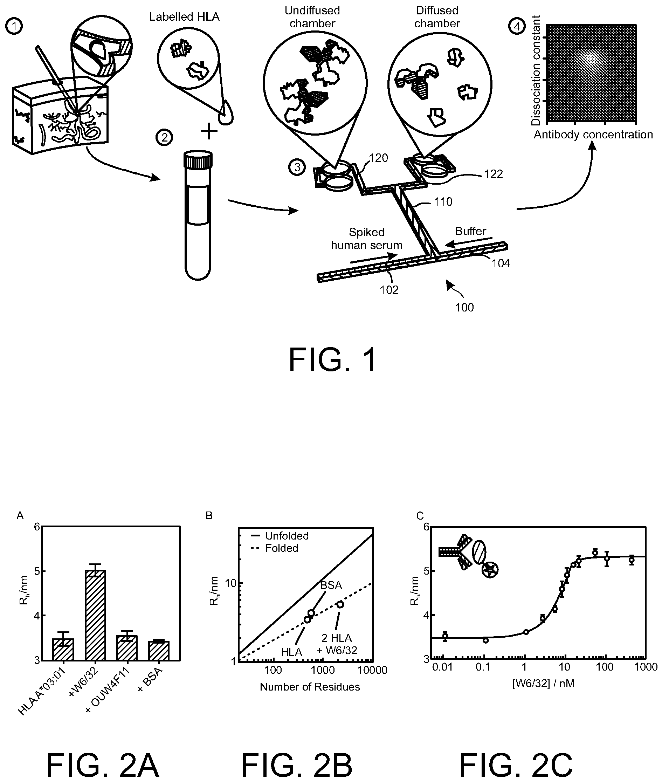

Referring to Figure 1, there is provided a schematic showing the apparatus and method of the present invention. Human serum sample is taken from a patient such as a human patient that is then incubated with labelled HLA. The sample is then introduced into a device **100**. The device **100** comprises a sample inlet channel **102** where the human serum sample is loaded onto the device and it also comprises an auxiliary inlet channel **104** for introducing a blank fluid flow. The sample fluid flow and the auxiliary fluid flows are introduced into a diffusional channel **110** to generate a laminar flow within the distribution channel **110**. The component or components, which may be biomolecules, are distributed between the sample and auxiliary flows within the distribution channel **110**. The distribution of the biomolecule occurs via on or more of diffusion, electrophoresis or thermophoresis.

The device **100** further comprises at least two outlet channels **120, 122** located downstream from the distribution channel **110** where the fluid flow comprising the sample and auxiliary flows in the distribution channel **110** is divided the fluid into two outlet flows, one flow entering each outlet channel **120, 122**. As shown in Figure 1, the distribution channel **110**, together with the sample inlet channel **102**, auxiliary inlet channel **104** and the outlet channels **120, 122** forms an H-filter.

A measurement module comprising a detector may be positioned at the outlet channels to detect the biomolecule of interest. The fluorescence at each of the outlet channels **120, 122** can be measured. From the ratio between the fluorescence in both the outlet channels, the hydrodynamic radius, Rh, of the protein can be determined. In some cases, the measurement module is able to detect and/or identify a signature profile of the biomolecule of interest to obtain a measured dataset of the detected biomolecule. The measured dataset can then be transmitted to an analysis module.

The size of the complex is subsequently determined by microfluidic diffusional sizing of which the dissociation constant $K_d$ and the antibody concentration are evaluated using Bayesian analysis.

MDS has been shown to allow the determination of binding parameters by utilising the measured hydrodynamic radius, Rh, of a fluorescently labelled protein by tracking its spatial and temporal evolution in a microfluidic channel under laminar flow as illustrated in Figure 1.

Referring to Figures 2A to C, there is shown in Figure 2A binding experiment of HLA A*03:01 using diffusional sizing. Every bar shows the average of triplicate measurements. A significant change in hydrodynamic radius indicates a positive binding event between HLA A*03:01 and W6/32. In contrast, no binding can be observed between HLA and OUW4F11 or BSA. Referring to Figure 2B, there is shown a correlation of hydrodynamic radii with the number of residues. The radii determined for HLA both free and bound to the antibody W6/32 agree well with the assumption of a folded protein.

Referring to Figure 2C, there is shown a binding curve of 25 nM HLA A*03:01 with varying concentrations of antibody W6/32. The points give the size measured for the labelled HLA species in varying fractions of unbound and unbound state, averaged over the data of at least three replicates, the curve is a least-square fit to a non-linear binding equation. From the fit, the dissociation constant $K_d$ = 961.3 $\pm$ 115.7 pM and the binding site concentration $B_0$ = 12.98 $\pm$ 1.00 nM could be determined, resulting in a stoichiometry of one antibody to two HLA molecules.

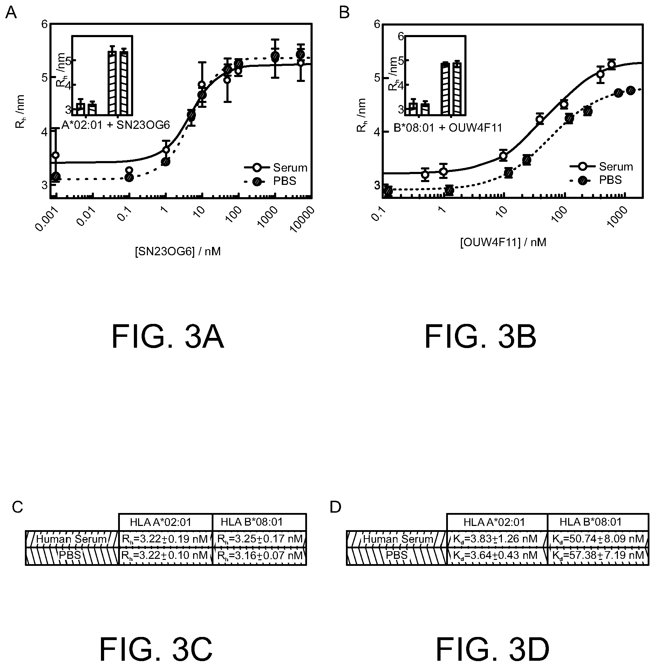

Referring to Figure 3A, there is shown a binding curve of 5 nM HLA A*02:01 against antibody SN23OG6 in human serum (red) and PBS (blue). From the fit, the $K_d$ = 3.83 + 1.26 nM in serum and $K_d$ = 3.64 $\pm$ 0.43 nM in PBS was determined, which perfectly agree with each other. Further, a binding ratio 1 to 2 for binding of 2 antigens per antibody is obtained. The inserted graph shows the increase of the Stokes radius (Rh) for HLA A*02:01 upon addition of the specific antibody SN23OG6.

Referring to Figure 3B, there is shown a binding curve of 1.2 nM HLA B*08:01 against antibody OUW4F11 in human serum (red) and PBS (blue). From the fit, $K_d$ = 50.74 $\pm$ 8.09 nM in serum and $K_d$ = 57.38 $\pm$ 7.19 nM in PBS and a binding ratio of $n = \dfrac{1}{2}$ was determined, showing consistency. The inserted graph shows the increase of the Stokes radius for HLA B*08:01 upon addition of the specific antibody OUW4F11.

Referring to Figure 3C, there is illustrated a summary of the hydrodynamic radii for the unbound antigens and in Figure 3D, there is shown the dissociation constants in human serum in comparison to their values in pure buffer, demonstrating consistent values under both buffer conditions.

In order to demonstrate the usability of the MDS assay for absolute quantification and characterisation of molecules in complex media, well characterised interactions were measured in human serum. First, varying concentration of antibodies SN23OG6 and OUW4F11 are added into blank human serum and measured their interactions with HLA A*02:01 and HLA B*08:01, respectively. As a first step, the hydrodynamic radii of both antigens are determined. The radii of pure HLA obtained human serum are consistent with the theoretical values as well as the radii obtained in buffer as shown in Figure 3C, suggesting applicability of the MDS assay for human serum.

This also shows that human serum does not contain any other protein that can perturb the measurements by binding to the investigated antigen. For these two antigen/antibody pairs, the dissociation constants determined are independent of the buffer conditions used. More specifically, for the interaction of the antibody SN23OG6 against HLA A*02:01, the dissociation constant $K_d$ = 3.83 $\pm$ 1.26 nM in human serum is in good agreement with the $K_d$ = 3.64 $\pm$ 0.43 nM in PBS (see Figure 3A. Similar can be observed for the antibody OUW4F1 against HLA B*08:01, with a $K_d$ = 50.74 $\pm$ 8.09 nM similar to $K_d$ = 57.38 $\pm$ 7.19 nM in PBS (see Figure 3B). The different saturation levels under the different media conditions lie within the error range, and are most likely caused by minor conformational variations between individual antigens.

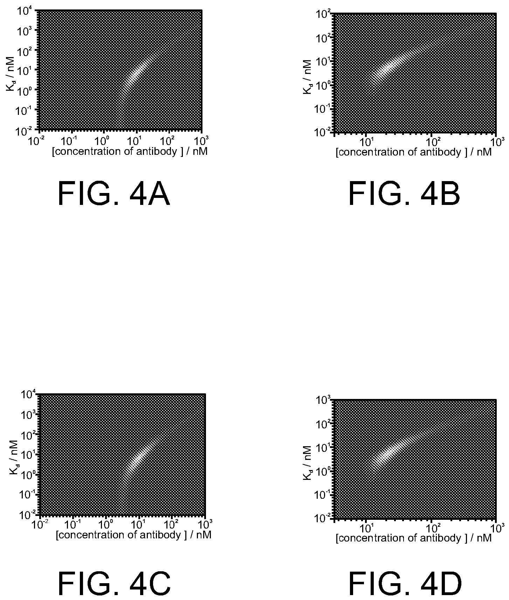

Referring to Figures 4A to 4D, there are shown data on testing of human patient serum against different HLA variants. By way of example, the size increase of both HLA A*02:01 and HLA A*24:02 can be an indication of protein interactions.

The hydrodynamic radius, Rh, of the measured species can be correlated with the molecular weight (Mw) of the biomolecule, which is then used to sum and examine whether the Rh values observed make sense as Rh can't be added since it is a volumetric measure and scales with molecular weight by approximately a one third power.

Referring to Figure 4A, there is shown Bayesian analysis of the binding interaction of HLA A*02:01 with HLA-specific antibody in patient serum. From this, an antibody concentration in the nanomolar range can be determined, with a maximal probability value 6.02 $\pm$ 2.66 nM. The $K_d$ is not well constrained, but has an upper limit $K_d \leq 30$ nM.

Referring to Figure 4B, there is shown Bayesian Analysis for the interaction of HLA A*24:02 with HLA-specific antibody in the serum of the same patient. Here, an antibody concentration of about 15.27 $\pm$ 0.24 nM is found, for the $K_d$ only an upper limit of $K_d \leq 3$ nM.

In some embodiments, the mean fluorescence intensities of a patient serum against HLA A*02:01 and A*24:01 at different serum concentration can be shown.

As shown in Figure 4C, there is shown the Bayesian analysis of the binding interactions of antibody at ten times dilution of Patient Serum against HLA A*02:01. As shown in Figure 4D, there is shown the Bayesian analysis of the binding interactions of antibody at ten times Dilution of Patient Serum against HLA A*24:02.

Subsequently, the applicability of this platform in patient serum is further described herein. For this purpose, the interaction of both HLA A*02:01 and HLA A*24:02 with the same patient serum is of interest. The patient can be highly sensitised for both HLA types, showing a higher Mean Fluorescence Intensity (MFI) on the Luminex platform for the HLA A*24:02. As shown in Figure 4A, a size increase can be observed under the given conditions, showing that binding occurs.

By using Bayesian analysis, both the affinity and the antibody concentration can be quantified. For the interaction of serum allo-antibody with HLA A*02:01, the concentration of the antibody could be constrained to $6.02 \pm 2.66$ nM, with a $K_d \leq 30$ nM, assuming a binding ratio of one to two. Similarly, for the same patient serum, antibody binding to HLA A*24:02 can be investigated, which show a higher Luminex signal than for HLA A*02:01. The antibody concentration could be determined as $15.26 \pm 3.17$ nM, and the affinity can be given with an upper limit of $K_d \leq 3$ nM.

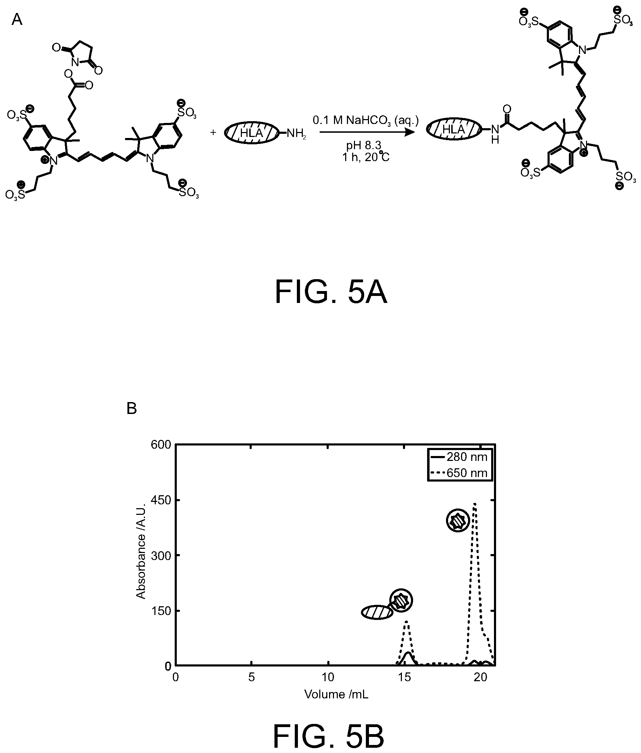

As shown in Figure 5A and 5B, there is shown a labelling strategy of the biomolecule of interest. Figure 5A refers to a reaction scheme and Figure 5B shows chromatogram on a Superdex 200 increase column from GE Healthcare with a flow rate of 0.5 mL/min and PBS as elution buffer. It is possible to deconvolute the signal into the fluorescence of interest and the background contribution.

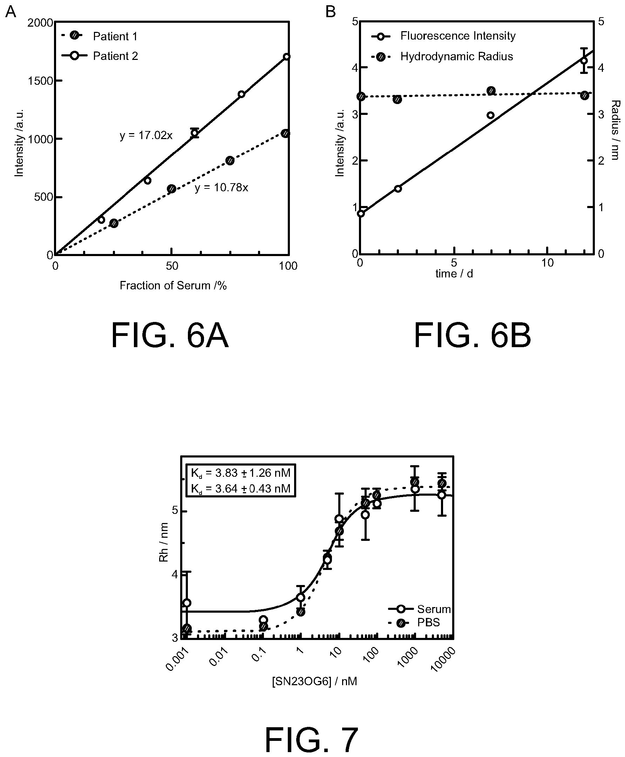

Referring to Figure 6A and Figure 6B, there are shown analyses of sample autofluorescence. As shown in Figure 6A, the fluorescence intensity increases with increasing serum concentration linearly, as expected. The absolute fluorescence intensity, thereby, differs between different patients. As shown in Figure 6B, the serum fluorescence Intensity increases over course of 12 days, whilst the radii of labelled biomolecules in serum does not change over the same time course.

Using the apparatus and method as disclosed in the present invention, it is possible to determine the hydrodynamic radius Rh in human serum. The auto-fluorescence of human serum above 600 nm seems to be reduced, although a signal is detectable on the microfluidic platform.

Referring to Figure 7, there is shown binding curve for the interaction between SN23OG6 against HLA A*02:01. The dissociation constants in both cases are consistent, and both have an equal stoichiometry of 1 to 2. It is possible to determine the stoichiometry and dissociation constant in antibody doped serum consistently with the buffer conditions.

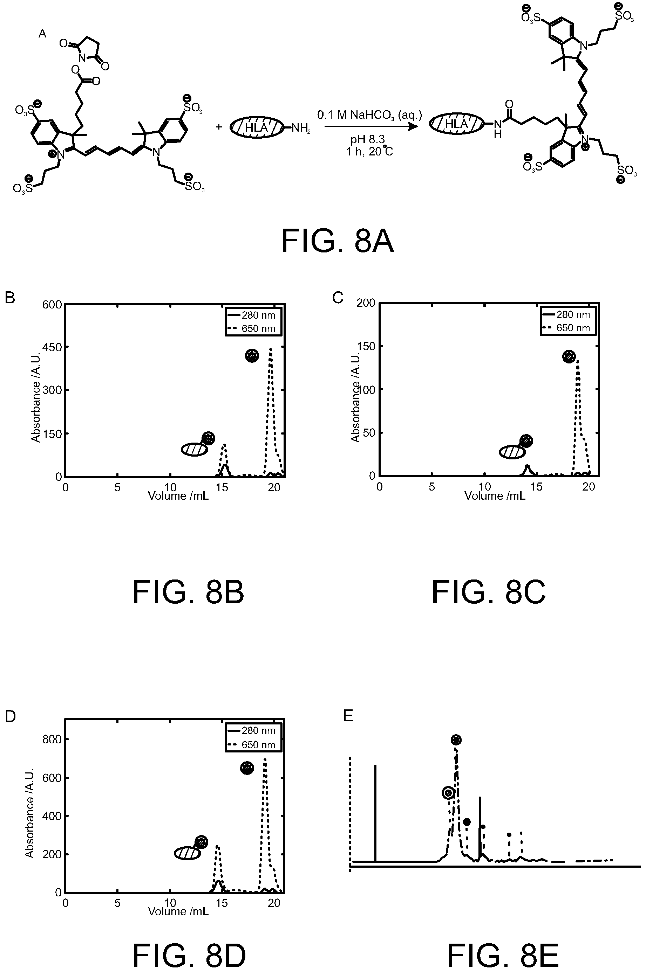

Figures 8A to 8E shows a schematic of covalent labelling strategy as disclosed herein. Figure 8A shows a reaction Mechanism of linking an amine on a protein to Alexafluor® 647 using an amide coupling. Figure 8B shows a chromatogram of the purification of HLA A*02:01. Figure 8C shows HLA A*03:01 and Figure 8D HLA B*08:01 after labelling, showing the elution of HLA in one fraction. Figure 8E shows a chromatogram for the purification of the commercially available streptavidin mixture.

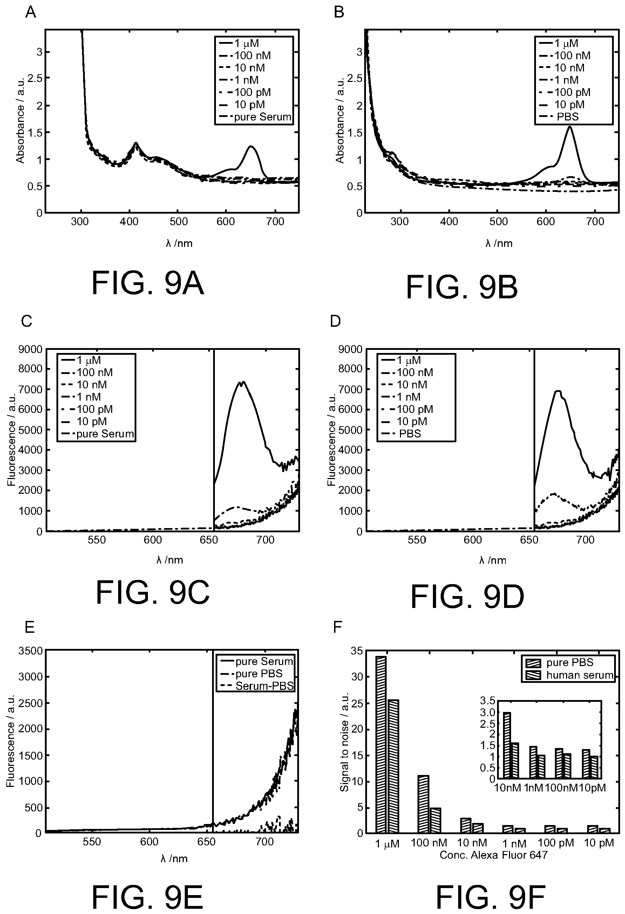

Figure 9A illustrates the determination of the Absorbance of varying concentration of Alexafluor® 647 in in human serum and Figure 9B in buffer, showing no increased absorbance around 663 nm in human serum. Figure 9C shows the fluorescence emission of Alexafluor® 647 in human serum and Figure 9D in PBS. Figure 9E shows a comparison of fluorescence emission of both human serum and PBS shows no difference. Figure 9F shows the signal to noise aspect ratios in human serum and in PBS. The signal-to-noise ratio is slightly reduced in human serum compared to PBS.

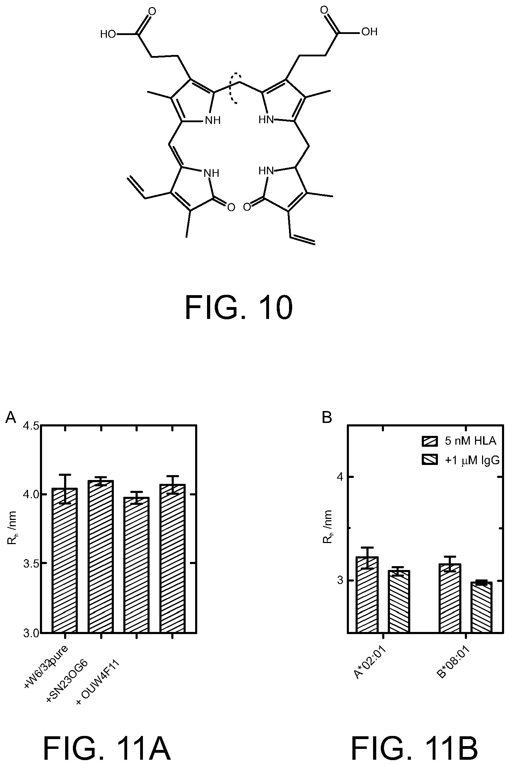

Figure 10 shows the structure of billirubin. The arrow indicates the rotation which is hindered by complexation of billirubin to HSA, a possible source of the fluorescence of the human serum.

Referring to Figures 11A to 11B, there is shown negative control experiments. Figure 11A shows a comparison of hydrodynamic radii of Alexafluor® 647 labelled BSA, both pure and after incubation with different antibodies. This demonstrates that the HLA specific antibodies do not recognise the fluorophore, thus, every binding interaction determined can be assumed specific. Figure 11B shows the hydrodynamic radii of different HLA variants determined purely or after incubation with 200 nM IgG (ab205198), showing no size increase and, thus, suggesting selective interaction between these HLA variants and specific alloantibodies.

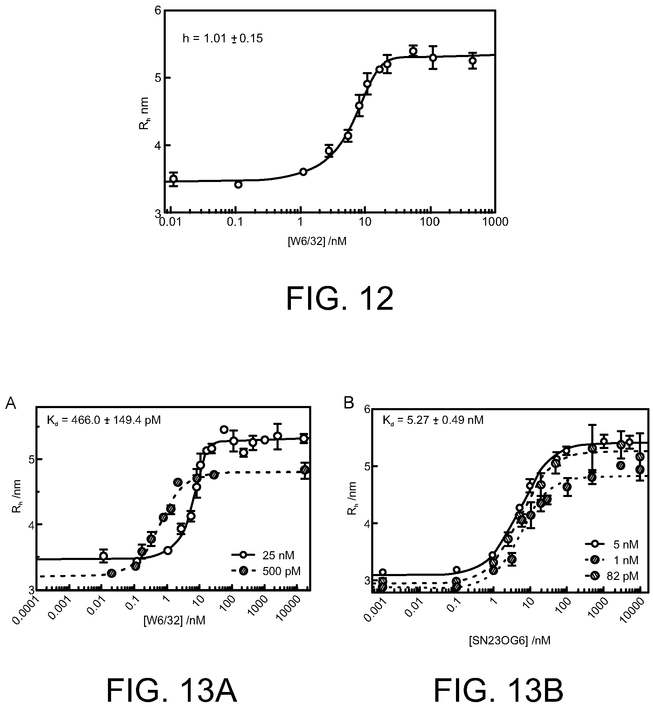

Figure 12 provides another negative control experiment. As shown in Figure 12, the binding curve of 25 nM HLA A*03:01 with varying concentration of antibody W6/32. The blue points are averaged over the data measured in three replicates; the red line is the fit according to a Hills equation. From this data, the Hills coefficient h = $1.01 \pm 0.15$ could be determined.

Referring to Figure 13A to 13B, there is shown global fit for binding curves. Figure 13A shows a global fit for the binding curves for the interaction between HLA A*03:01 and antibody W6/32 at 25 nM and 500 pM HLA A*03:01. From this fit, a $K_d = 466.0 \pm 149.4$ pM could be determined. Figure 13B shows a global fit for the interaction between HLA A*02:01 and SN23OG6 at 5 nM. 1nM and 82 pM HLA A*02:0. This yields a $K_d = 5.27 + 0.49$ nM.

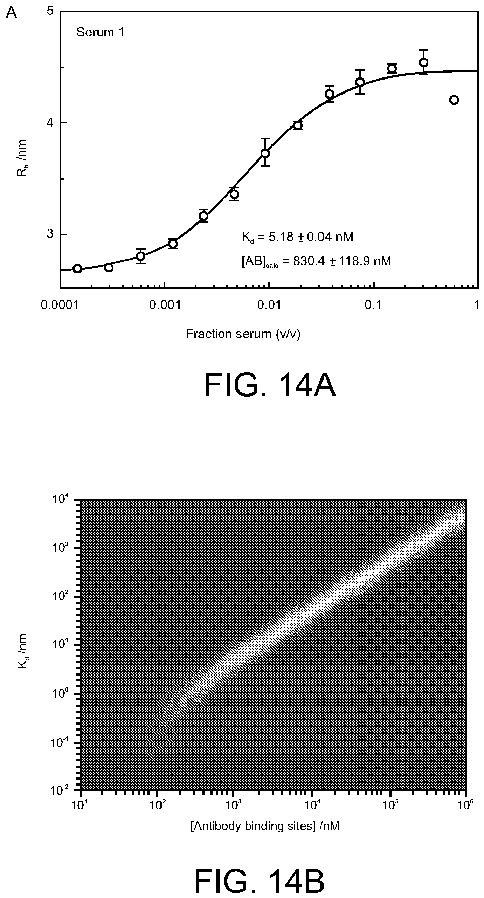

Referring to Figure 14A there is shown a binding curve of blinded human serum against 1 nM HLA A*02:01. An antibody concentration $[AB]_{cal} = 138.7 \pm 47.9$ and a $K_d = 961.3 \pm 115.7$ pM were obtained. After analysis, it was revealed that the blinded serum had 110 nM specific antibodies, showing the effectiveness in determining the $[AB]_{cal}$. Bayesian analysis of the blinded human serum is shown in Figure 14B. The Bayesian plot shows that the ratio between antibody concentration and $K_d$ is well contrained. Binding curve with non-linear fit and with Bayesian analysis can be measured.

**Analysis of MDS data**

The analysis of MDS data to establish a binding equilibrium between antibody and antigen are shown below.

Assuming 1:1 binding, we therefore have the following equilibrium

$$Ab + H \rightleftharpoons AbH \qquad (1)$$

where AbH denotes the bound antibody-HLA complex.

$$K_d = \frac{[Ab][H]}{[AbH]} \qquad (2)$$

Expressing the total concentrations of antibody and HLA as $[Ab]_0$ and $[H]_0$, respectively:

$$[AbH] = \frac{([Ab]_0 - [AbH])([H]_0 - [AbH])}{K_d} \qquad (3)$$

$$[AbH]^2 - ([Ab]_0 + [H]_0 + K_d)[AbH] + [Ab]_0[H]_0 = 0 \qquad (4)$$

Solving this:

$$[AbH] = \frac{[Ab]_0 + [H]_0 + K_D - \sqrt{([Ab]_0 + [H]_0 + K_D)^2 - 4[Ab]_0[H]_0}}{2} \qquad (5)$$

In the microfluidic diffusional sizing method, the measured hydrodynamic radius of our reaction mixture is calculated by recording the total fluorescence intensities of the 'diffused' and 'undiffused' channels, termed $I_d$ and $I_u$, respectively.

Since the measured radius is not linearly correlated with the concentrations of free and bound labelled HLA, in order to avoid skewing the data, the fraction of labelled HLA that ends up in the 'diffused' channel $I_d/I_d+I_u$ can be observed and used. In order to relate the observed quantity to the predicted concentration bound,[AbH], $\rho f$ and $\rho b$ are introduced as the fractions of free and bound HLA, respectively, that are detected in the 'diffused' channel. The following equations can be obtained as shown below:

$$I_d = \kappa([AbH]_{\rho_b} + ([H]_0 - [AbH])_{\rho_f}) \qquad (6)$$

$$I_u = \kappa\left([AbH](1 - \rho_b) + ([H]_0 - [AbH])(1 - \rho_f)\right) \qquad (7)$$

where $\kappa$ is a constant that relates the concentration of HLA to the fluorescence intensity observed. Therefore the predicted fraction to end up in the diffused channel, $f_d$ is:

$$f_d = \frac{([AbH]_{\rho_b} + ([H]_0 - [AbH])_{\rho_f})}{[H]_0} \qquad (8)$$

with [AbH] as determined in the above equation. Therefore, the predicted fraction to end up in the diffused channel is a function of $\rho f$, $\rho b$, $K_d$ and the total concentrations of antibody and HLA and this is denoted by $f_{d(\rho f, \rho b, K_d, [Ab]_0, [H]_0)}$.

Using $f_d$ as the observable, the $\rho_f$, $\rho_b$, and $K_d$ can therefore be determined as the unknown parameters by Bayesian inference as disclosed in further detail below. In order to account for the actual 1:2 non-cooperative binding stoichiometry of antibody:HLA, we assume that the radii of the singly and doubly bound antibody are equal, as their sizes are within the expected error of the experimental method. Therefore, we simply use [Ab] to denote concentration of antibody binding sites, rather than antibody concentration, and $K_d$ is thus the dissociation constant with respect to binding sites. As each IgG antibody contains two binding sites, we simply multiply antibody concentration by two to obtain [Ab].

**Determining unknown antibody concentration**

In patient serum samples, the concentration of antibody in serum is unknown. The concentration of HLA-reactive antibody binding sites in a given sample, $[Ab]_0$ is related to the concentration of binding sites in the original serum, $[Ab]_{tot}$, and the fraction of serum used in this particular sample, $\alpha$, by

$$[Ab]_0 = \alpha[Ab]_{tot} \qquad (9)$$

where $0 \leq \alpha < 1$.

Substituting this into equation 5 as above, an updated equation is shown below:

$$[AbH] = \frac{\alpha[Ab]_{tot}+[H]_0+K_D+\sqrt{(\alpha[Ab]_{tot}+[H]_0+K_D)^2-4a[Ab]_{tot}[H]_0}}{2} \qquad (10)$$

**Serum auto-fluorescence**

Using this expression in equation 8 we get a new expression for the predicted fraction to end up in the diffused channel, $f_d(\rho_f,\rho_b,K_d,[Ab]_{tot},\alpha,[H]_0)$.

A further complication of performing the measurements in human serum is serum auto-fluorescence, which requires pre-processing of the data. Since the raw channel fluorescence intensities are measured, this background fluorescence can be corrected for. By performing a calibration of the serum fraction and intensity in each channel in the absence of HLA, a linear relationship between the fluorescence intensity of each channel and the serum fraction, $\alpha$ can be obtained:

$$I_{d,s} = s_d\alpha \qquad (11)$$

$$I_{u,s} = s_u\alpha \qquad (12)$$

where $I_{d,s}$ and $I_{u,s}$ are the intensities in the 'diffused' and 'undiffused' channels arising from the serum, respectively, and $s_d$ and $s_u$ are constants obtained through linear regression.

A datapoint, $y$, is thus computed during pre-processing by

$$y = \frac{I_d-s_d\alpha}{(I_d+I_u)-\alpha(s_d+s_u)} \qquad (13)$$

It is therefore possible to analyse the data by Bayesian inference, with $\rho_f$, $\rho_b$, $K_d$, and $[Ab]_{tot}$ as unknown parameters, thereby obtaining values for both $K_d$, and $[Ab]_{tot}$ through binding measurements by microfluidic diffusional sizing.

**Bayesian inference**

The Bayesian inference analysis method utilises Bayes' theorem, and allows the determination of the probability distribution of unknown parameters, given the observed data, by the following equation

$$P(parameters|data) \propto P(parameters)P(data|parameters) \qquad (14)$$

where P(parameters|data) is known as the posterior, P(parameters) as the prior, and P(data|parameters) as the likelihood. The prior probability distribution is an expression of our information about the system before any measurement data can be acquired. For $\rho_f$ and $\rho_b$, prior is assumed to be flat in linear space, whereas for the $K_d$ and total concentration of antibody, a prior that is flat in logarithmic space is more appropriate, to reflect the scale invariance of the problem.

Experimental measurement data as described herein are normally to be distributed about the true value, and the likelihood function is therefore a Gaussian, centred on the theoretical measurement value.

$$P(data|parameters) \propto \exp\left[-\frac{1}{2\sigma^2}\sum_{i=1}^{N}\left(y_i - f_d\left(\rho_f, \rho_b, K_d, [Ab]_{tot}, \alpha_i, [H]_i\right)\right)^2\right] \quad (15)$$

where $f_d$ is defined in equation 8 with [AbH] defined in equation 10, $\alpha_i$ and $[H]_i$ are the concentrations of serum and antibody, respectively, in the $i^{th}$ measurement and yi is the pre-processed data point obtained in the $i^{th}$ measurement. In order to define an appropriate standard deviation, $\sigma$, for each dataset, the standard deviations of repeats of each measurement are calculated, and the maximum of these values are used as a global standard deviation for that dataset.

In order to maximise the information gained in each experiment, the entropy of the posterior distribution of the quantities of interest, e.g. the antibody concentration and affinity is used. The entropy of a distribution can simply be interpreted as a measure for how certain we are that a parameter had a specific value, the lower the entropy the more certain. Thus, the more a measurement decreases the entropy, the more information it contains. It can be predicted or estimated the entropy change if an additional measurement at a specific concentration of serum and antigen can be recorded. This is referred to as the expected entropy.

By calculating the expected entropy for all possible measurements i.e. all combinations of concentrations of serum and antigen, within the limits imposed by the experiment, the measurement point associated with the biggest expected decrease in entropy can be found. This can be performed in an iterative manner: after taking the first measurement, the expected entropy is calculated and the best next measurement is proposed. Once this measurement has been recorded, the expected entropies are updated and a new best next measurement is proposed. This process is repeated until the desired level of confidence and accuracy is obtained.

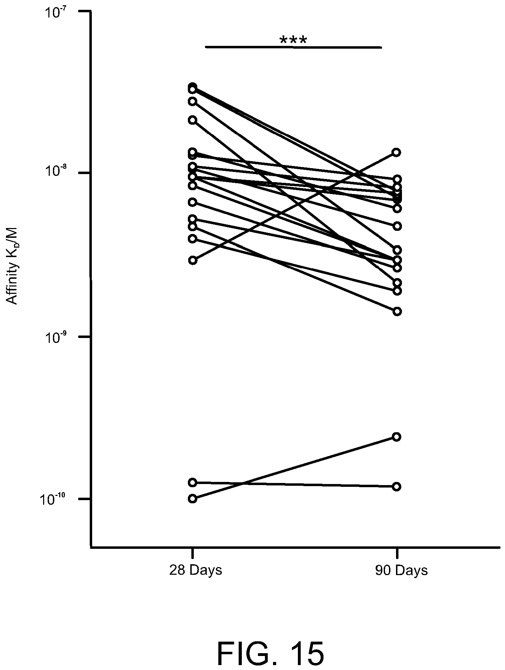

Referring to Figure 15, there is provided a plot that shows a microfluidic antibody affinity profiling (MAAP) of serum antibody reactivity against the Receptor Binding Domain (RBD) of SARS-CoV-2 Spike protein in patients with COVID-19. Sera from patients with COVID-19 are examined at 28 days and at 90 days after onset of symptoms. Patients with the full spectrum of COVID-19 severity, from asymptomatic to patients admitted in intensive care unit, were examined. MAAP analysis showed that affinities ($K_D$) of anti-RBD antibodies spanned over two orders of magnitude at both time points revealing a spectrum of humoral immunity against SARS-CoV-2. Moreover, MAAP analysis showed strengthening of the antibody response against SARS-CoV-2 within this time frame (reduction in $K_D$ at 3 months compared to the 1 month time point) in the majority of the patients examined, providing direct evidence of affinity maturation in patients recovering from COVID-19. The data as shown in Figure 15 demonstrates that MAAP analysis enables assessment of the magnitude and evolution of the humoral response directly in patient sera enabling further insights into the quality of the immune response after viral infection.

### Examples - Experimental details

### Determination of Auto fluorescence in Human Serum

Human serum (not containing specific anti-HLA antibodies) can be supplemented with PBS (pH 7.3, Oxoid tablets, Thermo Fisher Scientific Inc., Waltham, US; supplemented with NaN3 (0.02 % (w/v), Sigma Aldrich, St Louis, US)) and the fluorescent label Alexafluor® 647 (Thermo Fisher Scientific Inc., Waltham, US), to yield fluorophore concentrations between 10 pM and 1 $\mu$M in serum. Similar dilutions of fluorophore in buffer can be prepared for comparison. Subsequently, both absorption spectra and the emission spectra upon excitation at two wavelengths $\lambda_{ex,1}$ = 481 nm and $\lambda_{ex,2}$ = 632 nm can be recorded on a plate reader (Clariostar BMG Labtech, Ortenberg, DE).

### Labelling of HLA with Alexafluor® 647 fluorophore

To label HLA (different variants from Emory, Atlanta, US; in NaHCO3 (Sigma Aldrich, St Louis, US), 0.89 nmol, 1 equiv.), Alexa Fluor® 647 N-Hydroxysuccinimide ester (in DMSO 3 equiv.) can be added into a mixture containing HLA. The reaction mixture can be incubated for 1 hour at approx. 20°C, protected from light. The sample can be purified by size exclusion chromatography on a Superdex 200 increase 10/300 GL column (GE healthcare, Chicago, US) with a flow rate of approximately 0.5 mL/min and PBS (pH 7.3, supplemented with NaN3 (0.02% (w/v)) as eluent buffer, to yield labelled HLA (370 nM, DOL between 0.33 to 2.25, depending on variant). Conjugated HLA can then be stored at 4°C until further use.

### Microfluidic Diffusional Sizing (MDS)

All MDS experiments were performed using Fluidity One W (Fluidic Analytics, Cambridge, UK). The basic principle of MDS has been described elsewhere. In brief, labelled protein streams into the diffusion chamber from one side, auxiliary buffer from the other side. Due to the small channel size, laminar flow can be assumed, meaning that the particles can move into the buffer stream by diffusion only, whereby the rate depends on the size of the molecular complex. At the end of the diffusion channel or chamber, the stream is split again and the fluorescence at both sides of the chambers is measured. From the ratio between the fluorescence in both chambers, the hydrodynamic radius, Rh of the protein can be determined.

**Verification of Binding**

Labelled HLA A*03:01, A*02:01, and B*08:01, respectively, is diluted in PBS (pH 7.3, supplemented with NaN3 (0.02 % (w/v)) to yield a 5 nM solution. The size of the HLA conjugates can be determined by MDS. Similarly, labelled HLA variants and respective antibodies are mixed, to yield a 5 nM concentration of HLA with 1 $\mu$M antibody. These conjugates were incubated at 4°C for approx. 1 hour, then heated up to room temperature for 5 min and sized by MDS.

**Negative Control Experiments**

Labelled HLA variants were mixed with human IgG ab205198 (abcam, Cambridge, UK) or bovine serum albumin (BSA; Sigma Aldrich, St Louis, DE) to yield a concentration of 5 nM of HLA variants and 1 $\mu$M antibody. These conjugates are incubated at 4°C for 1 hour, then heated up to room temperature for 5 min and sized by MDS. Similarly, Alexafluor® 647 labelled BSA is diluted in PBS, to yield a solution of 5 nM of labelled BSA, as well as 250 equivalent of antibody, to demonstrate absence of unspecific interactions between the antibody and the fluorophore.

**Measurements in PBS**

Labelled HLA of a particular variant, together with a varying concentration of the antibody of interest, were added and diluted in PBS. The samples are incubated at 4°C for approx. 1 hour or at room temperature for approx. 30 min. Subsequently, the size was determined by MDS. The data is fitted according to the non-linear binding equation (equation 16), which relates the measured hydrodynamic radius, $R_{h,m}$, to the total concentrations of antibody, $[Ab]_0$, the binding site concentration, $[B]_0$, the dissociation constant $K_d$, the stoichiometric ratio, n, the total increase in the hydrodynamic radius, $\Delta R_{h,tot}$ and the hydrodynamic radius of unbound antigen, $R_{h,0}$, using GraphPad Prism (Version 8.2).

$$R_{h,m} = \left[ \left( \frac{[Ab]_0 + n[B]_0 + K_d}{2} - \sqrt{\frac{([Ab]_0 + n[B]_0 + K_d)^2}{4} - n[B]_0[Ab]_0} \right) \frac{\Delta R_{h,tot}}{n[B]_0} \right] + R_{h,0} \qquad (16)$$

For the fitting of the data to a Hill's equation 17 is used as shown below:

$$R_{h,m} = \left[ \left( \frac{[Ab]_0^h + [B]_0 + K_d^h}{2} - \sqrt{\frac{([Ab]_0^h + [B]_0 + K_d^h)^2}{4} - [B]_0[Ab]_0^h} \right) \frac{\Delta R_{h,tot}}{[B]_0} \right] + R_{h,0} \qquad (17)$$

As, experimentally, a non-cooperativity of HLA to antibody binding has been validated, a binding ration of n = 1/2 for 2 to 1 binding has been used further on in equation 16.

**Binding Measurements in Human Serum**

A range of different HLA types can be added to human serum, supplemented with varying concentrations of specific antibodies. The samples are incubated at room temperature (r.t.) for 30 min and subsequently, the size is determined by MDS. The data is fitted according to equation 18, using GraphPad Prism (Version 8.2).

$$R_{h,m} = R_{h,HLA} \cdot \left( 1 - \frac{qf + p}{mf + n} \right) + \left( \frac{qf + p}{mf + n} \right) \cdot R_{h,bg} \qquad (18)$$

**Bayesian Analysis**

The priors used for the radius of the free species, rf, and the radius of the bound species, rb, are flat in linear spice, while a flat log-space prior is used for $K_d$ and binding site concentration, meaning that the probability of the $K_d$ being between 1 nM and 10 nM equals the probability of lying between 10 nM and 100 nM. A flat log-space allows facilitates the constraint the order of magnitude.

Various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

Unless context dictates otherwise, the descriptions and definitions of the features set out above are not limited to any particular aspect or embodiment of the invention and apply equally to all aspects and embodiments which are described.

It will further be appreciated by those skilled in the art that although the invention has been described by way of example with reference to several embodiments. It is not limited to the disclosed embodiments and that alternative embodiments could be constructed without departing from the scope of the invention as defined in the appended claims.

**Claims**

1. An apparatus for characterising a biomolecule, the apparatus comprising

   a sample inlet channel configured to introduce a sample fluid including the biomolecule to the apparatus;
   an auxiliary inlet channel configured to introduce an auxiliary fluid to the apparatus;
   a distribution channel in fluid communication with the sample inlet channel and the auxiliary inlet channel; wherein the distribution channel is adapted to generate a lateral distribution of the biomolecule;
   a measurement module configured to detect a signature profile of the biomolecule to obtain a measured dataset of the detected biomolecule;
   wherein the measured dataset includes one or more of the following: auto fluorescence background measurements, flow profile, concentration, stoichiometry interactions, molecular weight of interacting biomolecules, total ligand concentration, binding sites,
   size, charge, modification such as post translational modification and/or the hydrodynamic radius;
   a storage location configured to store and maintain a stored dataset comprising a plurality of parameters that are associated with the measured dataset obtained from the measurement module; and
   an analysis module configured to receive the stored dataset from the storage location and correlate the stored dataset with the measured dataset from the measurement module to provide a correlation value,
   wherein the analysis module is further configured to use the correlation value to determine at least two characteristics of the biomolecule simultaneously using Bayesian analysis; and
   wherein the at least two characteristics are the concentration and the affinity or avidity of the biomolecule.

2. The apparatus according claim 1 wherein the apparatus further comprises a controller configured to receive the stored dataset from the storage location and to receive the measured dataset from the analysis module to further tune one or more parameters associated with the measured dataset; and
   an output module associated with the controller configured to provide a notification to an operator indicating a further cycle of measurements such that further measurements obtained provide a pre-determined level of confidence in the determined characteristics of the biomolecule.

3. The apparatus according to any one of the preceding claims, wherein the apparatus further comprises at least two outlet channels to divide the fluid in the distribution channel into two or more flows.

4. The apparatus according to any one of the preceding claims, wherein the analysis module is further configured to detect and determine the stoichiometry of each biomolecule.

5. The apparatus according to any one of the preceding claims, wherein the distribution channel is a T-sensor.

6. The apparatus according to any one of the preceding claims, wherein the biomolecule is labelled with a fluorophore, a quantum dot or a nanoparticle.

7. The apparatus according to any one of the preceding claims, wherein the fluorophore selected for labelling the biomolecule is in the far-red spectral region.

8. The apparatus according to any one of the preceding claims, wherein an electrode is provided at the upstream of the distribution channel and an electrode provided at the downstream of the distribution channel, configured to apply an electric field across the distribution channel.

9. The apparatus according to any one of the preceding claims, wherein the biomolecule is an antibody or an antibody fragment thereof, a polypeptide, a polynucleotide or a polysaccharide.

10. The apparatus according to any one of the preceding claims, wherein the antibody is an allo-antibody.

11. The apparatus according to any one of the preceding claims, wherein the biomolecule is a multi-biomolecule mixture.

12. The apparatus according to any one of the preceding claims, wherein the multi-biomolecule mixture comprises an antibody and an antigen, wherein the antigen is labelled.

**Patentansprüche**

1. Vorrichtung zum Charakterisieren eines Biomoleküls, die Vorrichtung umfassend einen Probeneinlasskanal, der konfiguriert ist, um ein Probenfluid, das das Biomolekül beinhaltet, in die Vorrichtung einzuleiten;

einen Hilfseinlasskanal, der konfiguriert ist, um ein Hilfsfluid in die Vorrichtung einzuleiten; einen Verteilerkanal in Fluidverbindung mit dem Probeneinlasskanal und dem Hilfseinlasskanal; wobei der Verteilerkanal angepasst ist, um eine seitliche Verteilung des Biomoleküls zu erzeugen; ein Messmodul, das konfiguriert ist, um ein Signaturprofil des Biomoleküls zu erfassen, um einen gemessenen Datensatz des erfassten Biomoleküls zu erlangen; wobei der gemessene Datensatz eines oder mehrere von Folgenden beinhaltet: Autofluoreszenz-Hintergrundmessungen, Strömungsprofil, Konzentration, stöchiometrische Wechselwirkungen, Molekulargewicht der wechselwirkenden Biomoleküle, Ligandenkonzentration insgesamt, Bindungsstellen, Größe, Ladung, Modifikation wie beispielsweise posttranslationale Modifikation und/oder den hydrodynamischen Radius;
einen Speicherort, der konfiguriert ist, um einen gespeicherten Datensatz, umfassend eine Vielzahl von Parametern, die mit dem gemessenen Datensatz assoziiert sind, der von dem Messmodul erlangt wird, zu speichern und beizubehalten; und
ein Analysemodul, das konfiguriert ist, um den gespeicherten Datensatz von dem Speicherort zu empfangen und den gespeicherten Datensatz mit dem gemessenen Datensatz von dem Messmodul zu korrelieren, um einen Korrelationswert bereitzustellen,
wobei das Analysemodul ferner konfiguriert ist, um den Korrelationswert zu verwenden, um mindestens zwei Charakteristiken des Biomoleküls gleichzeitig unter Verwendung von Bayes'scher Analyse zu bestimmen; und wobei die mindestens zwei Charakteristiken die Konzentration und die Affinität oder Avidität des Biomoleküls sind.

2. Vorrichtung nach Anspruch 1, wobei die Vorrichtung ferner eine Steuerung umfasst, die konfiguriert ist, um den gespeicherten Datensatz von dem Speicherort zu empfangen und den gemessenen Datensatz von dem Analysemodul zu empfangen, um einen oder mehrere Parameter, die mit dem gemessenen Datensatz assoziiert sind, weiter abzustimmen; und
ein mit der Steuerung assoziiertes Ausgangsmodul, das konfiguriert ist, um einem Bediener eine Benachrichtigung bereitzustellen, die einen weiteren Zyklus von Messungen angibt, sodass weitere erlangte Messungen ein vorbestimmtes Maß an Vertrauen in die bestimmten Charakteristiken des Biomoleküls bereitstellen.

3. Vorrichtung nach einem der vorhergehenden Ansprüche, wobei die Vorrichtung ferner mindestens zwei Auslasskanäle umfasst, um das Fluid in dem Verteilerkanal in zwei oder mehrere Ströme aufzuteilen.

4. Vorrichtung nach einem der vorhergehenden Ansprüche, wobei das Analysemodul ferner konfiguriert ist, um die Stöchiometrie von jedem Biomolekül zu erfassen und zu bestimmen.

5. Vorrichtung nach einem der vorhergehenden Ansprüche, wobei der Verteilerkanal ein T-Sensor ist.

6. Vorrichtung nach einem der vorhergehenden Ansprüche, wobei das Biomolekül mit einem Fluorophor, einem Quantenpunkt oder einem Nanopartikel markiert ist.

7. Vorrichtung nach einem der vorhergehenden Ansprüche, wobei das Fluorophor, das zur Markierung des Biomoleküls ausgewählt ist, in dem fernroten Spektralbereich ist.

**8.** Vorrichtung nach einem der vorhergehenden Ansprüche, wobei eine Elektrode stromaufwärts von dem Verteilerkanal und eine Elektrode stromabwärts von dem Verteilerkanal bereitgestellt ist, die konfiguriert sind, um ein elektrisches Feld an dem Verteilerkanal anzulegen.

**9.** Vorrichtung nach einem der vorhergehenden Ansprüche, wobei das Biomolekül ein Antikörper oder ein Antikörperfragment davon, ein Polypeptid, ein Polynukleotid oder ein Polysaccharid ist.

**10.** Vorrichtung nach einem der vorhergehenden Ansprüche, wobei der Antikörper ein Allo-Antikörper ist.

**11.** Vorrichtung nach einem der vorhergehenden Ansprüche, wobei das Biomolekül ein Multi-Biomolekül-Gemisch ist.

**12.** Vorrichtung nach einem der vorhergehenden Ansprüche, wobei das Multi-Biomolekül-Gemisch einen Antikörper und ein Antigen umfasst, wobei das Antigen markiert ist.

**Revendications**

**1.** Appareil destiné à caractériser une biomolécule, l'appareil comprenant un canal d'entrée d'échantillon configuré pour introduire un fluide échantillon comprenant la biomolécule dans l'appareil ;

un canal d'entrée auxiliaire configuré pour introduire un fluide auxiliaire dans l'appareil ;
un canal de distribution en communication fluidique avec le canal d'entrée d'échantillon et le canal d'entrée auxiliaire ; ledit canal de distribution étant adapté pour générer une distribution latérale de la biomolécule ;
un module de mesure configuré pour détecter un profil de signature de la biomolécule afin d'obtenir un ensemble de données mesuré de la biomolécule détectée ;
ledit ensemble de données mesuré comprenant un ou plusieurs des éléments suivants : des mesures de fond d'autofluorescence, un profil d'écoulement, une concentration, des interactions de stoechiométrie, un poids moléculaire des biomolécules en interaction, une concentration totale de ligand, des sites de liaison, une taille, une charge, une modification telle qu'une modification post-translationnelle et/ou le rayon hydrodynamique ;
un emplacement de stockage configuré pour stocker et conserver un ensemble de données stocké comprenant une pluralité de paramètres qui sont associés à l'ensemble de données mesuré obtenu à partir du module de mesure ; et
un module d'analyse configuré pour recevoir l'ensemble de données stocké à partir de l'emplacement de stockage et corréler l'ensemble de données stocké avec l'ensemble de données mesuré à partir du module de mesure pour fournir une valeur de corrélation,
ledit module d'analyse étant en outre configuré pour utiliser la valeur de corrélation pour déterminer au moins deux caractéristiques de la biomolécule simultanément à l'aide d'une analyse bayésienne ; et
lesdites au moins deux caractéristiques étant la concentration et l'affinité ou l'avidité de la biomolécule.

**2.** Appareil selon la revendication 1, ledit appareil comprenant en outre un dispositif de commande configuré pour recevoir l'ensemble de données stocké en provenance de l'emplacement de stockage et pour recevoir l'ensemble de données mesuré en provenance du module d'analyse pour régler en outre un ou plusieurs paramètres associés à l'ensemble de données mesuré ; et
un module de sortie associé au dispositif de commande configuré pour fournir une notification à un opérateur indiquant un cycle de mesures supplémentaire de sorte que des mesures supplémentaires obtenues fournissent un niveau de confiance prédéfini dans les caractéristiques déterminées de la biomolécule.

**3.** Appareil selon l'une quelconque des revendications précédentes, ledit appareil comprenant en outre au moins deux canaux de sortie pour diviser le fluide dans le canal de distribution en deux flux, ou plus.

**4.** Appareil selon l'une quelconque des revendications précédentes, ledit module d'analyse étant en outre configuré pour détecter et déterminer la stoechiométrie de chaque biomolécule.

**5.** Appareil selon l'une quelconque des revendications précédentes, ledit canal de distribution étant un capteur T.

**6.** Appareil selon l'une quelconque des revendications précédentes, ladite biomolécule étant marquée avec un fluorophore, un point quantique ou une nanoparticule.

7. Appareil selon l'une quelconque des revendications précédentes, ledit fluorophore sélectionné pour marquer la biomolécule se trouvant dans la région spectrale du rouge lointain.

8. Appareil selon l'une quelconque des revendications précédentes, une électrode étant prévue en amont du canal de distribution et une électrode étant prévue en aval du canal de distribution, configurées pour appliquer un champ électrique à travers le canal de distribution.

9. Appareil selon l'une quelconque des revendications précédentes, ladite biomolécule étant un anticorps ou un fragment d'anticorps de celui-ci, un polypeptide, un polynucléotide ou un polysaccharide.

10. Appareil selon l'une quelconque des revendications précédentes, ledit anticorps étant un allo-anticorps.

11. Appareil selon l'une quelconque des revendications précédentes, ladite biomolécule étant un mélange multibiomolécule.

12. Appareil selon l'une quelconque des revendications précédentes, ledit mélange multibiomolécule comprenant un anticorps et un antigène, ledit antigène étant marqué.

**FIG. 1**

**FIG. 2A**　　　　**FIG. 2B**　　　　**FIG. 2C**

FIG. 3A

FIG. 3B

| | HLA A*02:01 | HLA B*08:01 |
|---|---|---|
| Human Serum | $R_h$=3.22±0.19 nM | $R_h$=3.25±0.17 nM |
| PBS | $R_h$=3.22±0.10 nM | $R_h$=3.16±0.07 nM |

FIG. 3C

| | HLA A*02:01 | HLA B*08:01 |
|---|---|---|
| Human Serum | $K_d$=3.83±1.26 nM | $K_d$=50.74±8.09 nM |
| PBS | $K_d$=3.64±0.43 nM | $K_d$=57.38±7.19 nM |

FIG. 3D

FIG. 4A

FIG. 4B

FIG. 4C

FIG. 4D

FIG. 5A

FIG. 5B

FIG. 6A

FIG. 6B

FIG. 7

FIG. 8A

FIG. 8B

FIG. 8C

FIG. 8D

FIG. 8E

FIG. 9A

FIG. 9B

FIG. 9C

FIG. 9D

FIG. 9E

FIG. 9F

# FIG. 10

# FIG. 11A

# FIG. 11B

FIG. 12

FIG. 13A

FIG. 13B

FIG. 14A

FIG. 14B

FIG. 15